(19) Europäisches Patentamt
European Patent Office
Office européen des brevets

(11) **EP 3 205 643 A1**

(12) **EUROPÄISCHE PATENTANMELDUNG**

(43) Veröffentlichungstag:
16.08.2017  Patentblatt 2017/33

(51) Int Cl.:
*C07C 319/18* (2006.01)    *C07C 323/22* (2006.01)

(21) Anmeldenummer: 16155700.4

(22) Anmeldetag: **15.02.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
• **RAUTENBERG, Stephan**
  **53332 Bornheim (DE)**

• **CEYLAN, Sascha**
  **63457 Hanau (DE)**
• **KÖRFER, Martin**
  **63796 Kahl (DE)**
• **HIEROLD, Judith**
  **30161 Hannover (DE)**
• **JAKOB, Harald**
  **63594 Hasselroth (DE)**
• **KAISER, Christian**
  **63857 Waldaschaff (DE)**
• **MALZKORN, Rainer**
  **63538 Großkrotzenburg (DE)**
• **MERKER, Thorsten**
  **63456 Hanau (DE)**
• **NORDSCHILD, Anja**
  **61440 Oberursel (DE)**

(54) **VERFAHREN ZUR HERSTELLUNG VON 3-METHYLTHIOPROPIONALDEHYD**

(57)  Die vorliegende Erfindung betrifft ein Verfahren zur Herstellung von 3-Methylthiopropionaldehyd durch Umsetzung von Methylmercaptan mit Acrolein, bei dem Abweichungen in der Stöchiometrie von Methylmercaptan zu Acrolein in der Umsetzung zu 3-Methylthiopropionaldehyd durch Zufuhr oder durch Bildung von 1,3-Bis(methylthio)-1-propanol ausgeglichen werden, sowie die Verwendung von 1,3-Bis(methylthio)-1-propanol als stabile Lagerform für Methylmercaptan und/oder 3-Methylthiopropionaldehyd

EP 3 205 643 A1

**Beschreibung**

Die vorliegende Erfindung betrifft ein Verfahren zur Herstellung von 3-Methylthiopropionaldehyd durch Umsetzung von Methylmercaptan mit Acrolein, bei dem die Abweichungen in der Stöchiometrie der Reaktanden durch Zufuhr oder durch Bildung von 1,3-Bis(methylthio)-1-propanol ausgeglichen werden, sowie die Verwendung von 1,3-Bis(methylthio)-1-propanol als stabile Lagerform für Methylmercaptan und/oder 3-Methylthiopropionaldehyd.

3-Methylthiopropionaldehyd, auch bekannt unter der Abkürzung MMP für Methylmercaptopropionaldehyd oder unter dem Namen 4-Thiapentanal (UN-Nummer 2785), ist ein wichtiges Intermediat in der Herstellung von D,L-Methionin und seinem Hydroxy-Analogen 2-Hydroxy-4-methylthiobutter-säure, auch bekannt unter der Abkürzung MHA für Methionin-Hydroxy-Analog. Die Herstellung von 3-Methylthiopropionaldehyd durch Umsetzung von Methylmercaptan mit Acrolein ist aus dem Stand der Technik bekannt. Aus der Literatur ist ebenfalls bekannt, dass diese Reaktion exotherm verläuft. Diese Wärmetönung ist insbesondere mit Blick auf Acrolein problematisch, da diese Verbindung leicht einer radikalischen oder ionischen Polymerisation unterliegt. Auch das Reaktionsprodukt 3-Methylthiopropionaldehyd selber unterliegt leicht Nebenreaktionen, die zur Bildung von hoch-siedenden Destillationsrückständen führt. Um die Wärmetönung bei der Herstellung von 3-Methyl-thiopropionaldehyd besser kontrollieren zu können, offenbart die DE 1618884 A ein zweistufiges Verfahren zur Herstellung von 3-Methylthiopropionaldehyd bei dem in der ersten Stufe Methylmercaptan mit 3-Methylthiopropionaldehyd umsetzt, bis die Wärmeentwicklung praktisch beendet ist, und das in der ersten Stufe erhaltene Reaktionsprodukt in der zweiten Stufe mit Acrolein umsetzt. Laut der DE 1618884 A wird die Umsetzung in der zweiten Stufe mit einem Überschuss von Acrolein durchgeführt.

In den Verfahren gemäß den Dokumenten DE 1618879 A und DE 1618889 A wird 3-Methyl-thiopropionaldehyd jeweils durch direkte Umsetzung von Methylmercaptan mit Acrolein hergestellt. Im Verfahren gemäß der technischen Lehre der DE 1618889 A wird 3-Methylthiopropionaldehyd durch Umsetzung von Methylmercaptan mit einer Acrolein und Wasser enthaltenden Lösung hergestellt, die durch Kondensation oder Wasserbehandlung der Reaktionsgase aus der katalytischen Oxidation von Propylen erhalten wurde, wobei die Acrolein enthaltende Lösung einen Wassergehalt von weniger als 15 Gewichtsprozent aufweist und frei von ungesättigten organischen Säuren ist. Im Verfahren gemäß der technischen Lehre der DE 1618879 A wird 3-Methylthiopropionaldehyd durch Umsetzung von Acrolein mit Methylmercaptan in flüssiger Phase bei Temperaturen bis 120°C hergestellt, wobei die Rektifikation des erhaltenen rohen 3-Methylthiopropionaldehyd bei spezifischen Temperaturen und für eine spezifische Dauer durchgeführt wird. Sowohl die DE 1618879 A als auch die DE 1618889 A lehrt die Verwendung eines Überschusses von Acrolein zu Methylmercaptan in der Herstellung von 3-Methylthiopropionaldehyd.

Methylmercaptan und Acrolein reagieren in einem äquimolaren Verhältnis zu 3-Methylthiopropionaldehyd. Jede Abweichung von dieser Stöchiometrie führt daher entweder zu einem Überschuss von Acrolein oder von Methylmercaptan. Bei einem Überschuss von Acrolein unterliegt das überschüssige Acrolein sehr leicht einer Polymerisation. Denn Acrolein kann sowohl kationisch oder anionisch als auch radikalisch polymerisieren. Die Bildung der Polymere erfolgt außerdem irreversibel, und daher kann das polymerisierte Acrolein nicht wieder für eine Umsetzung mit Methylmercaptan zurückgewonnen werden. Die Verwendung eines Überschusses von Acrolein in der Synthese von 3-Methylthiopropionaldehyd gemäß der technischen Lehre der vorstehend genannten Dokumente hat daher den großen Nachteil, dass beträchtliche Mengen der teuren Ausgangsverbindung Acrolein durch Nebenproduktbildung irreversibel verloren gehen. Ein weiterer Nachteil des Stands der Technik besteht darin, dass die bei der Polymerisation von Acrolein gebildeten Nebenprodukte hauptsächlich höhersiedende Rückstände sind. Diese Hochsieder stören die nachfolgende Herstellung von Methionin oder MHA, z.B. bei der Kristallisation von Methionin. Daher muss das 3-Methylthiopropionaldehyd enthaltende Produktgemisch einer VakuumDestillation bei hohen Temperaturen unterworfen werden. Höhere Konzentrationen an höhersiedenden Rückständen führen allerdings zu Verlust an Wertstoffprodukten und müssen zudem kostenaufwendig entsorgt werden. Daher sind die aus dem Stand der Technik bekannten Verfahren nicht für die großtechnische wirtschaftliche Herstellung von Methionin geeignet.

Theoretisch könnte einem Überschuss von Acrolein in dem Reaktionsgemisch durch Zufuhr von Methylmercaptan begegnet werden. Allerdings gestaltet sich die Abtrennung von Methylmercaptan aus einem 3-Methylthiopropionaldehyd enthaltenden Gemisch schwieriger als gedacht. Beispielsweise lassen sich geringe Mengen von Methylmercaptan nur schwer mittels Destillation aus dem Produktgemisch entfernen. Folglich gehen auch in diesem Fall entsprechende Mengen des wertvollen Ausgangsstoffes Methylmercaptan verloren. Problematischer ist jedoch dass das im 3-Methylthiopropionaldehyd enthaltene Methylmercaptan bei der weiteren Umsetzung zum Methionin ausgast und dadurch die Methionin-Herstellung stört. Zudem führt das Ausgasen von Methylmercaptan zu einer erheblichen Geruchsbelästigung.

Ferner wäre es theoretisch auch möglich, Acrolein mit Methylmercaptan in den stöchiometrisch erforderlichen Mengen zu 3-Methylthiopropionaldehyd umzusetzen, beispielsweise unter Regulierung der freigesetzten Reaktionswärme. In großtechnischen kontinuierlich betriebenen Verfahren ist es in der Praxis jedoch nicht möglich, stöchiometrische Mengen der Reaktanden der betreffenden Umsetzung zuzuführen ohne die Substanzen in Lagerbehältern zu puffern bzw. zwischenzulagern. Dem Gefährdungspotential der zu lagernden Substanzen muss jedoch mit aufwendigen und kostenintensiven konstruktiven und sicherheitstechnischen Maßnahmen begegnet werden.

Es bestand daher ein Bedarf an einem Verfahren zur Herstellung von 3-Methylpropionaldehyd aus Acrolein und Methylmercaptan, das bei Schwankungen in den Mengen der zugeführten Ausgangsverbindungen Acrolein und Methylmercaptan Verluste von Methylmercaptan und die Bildung von Nebenprodukten durch Polymerisation von Acrolein vermieden werden.

Gelöst wird diese Aufgabe erfindungsgemäß dadurch, dass das durch Addition von Methylmercaptan an 3-Methylthiopropionaldehyd gebildete Hemithioacetal 1,3-Bis(methylthio)-1-propanol oder die Mischung des Hemithioacetals mit 3-Methylthiopropionaldehyd dazu verwendet wird, Abweichungen in der Stöchiometrie von Methylmercaptan zu Acrolein in der Umsetzung von Acrolein mit Methylmercaptan zu 3-Methylthiopropionaldehyd auszugleichen.

Gegenstand der vorliegenden Erfindung ist daher ein Verfahren zur Herstellung von 3-Methylthiopropionaldehyd durch Umsetzung von Methylmercaptan mit Acrolein dadurch gekennzeichnet, dass Abweichungen in der Stöchiometrie von Methylmercaptan zu Acrolein in der Umsetzung zu 3-Methylthiopropionaldehyd durch Zufuhr oder durch Bildung von 1,3-Bis(methylthio)-1-propanol ausgeglichen werden, wobei

wenn Methylmercaptan im Überschuss zu Acrolein vorliegt, überschüssiges Methylmercaptan mit 3-Methylthiopropionaldehyd zu 1,3-Bis(methylthio)-1-propanol umgesetzt wird,

oder

wenn Acrolein im Überschuss zu Methylmercaptan vorliegt, 1,3-Bis(methylthio)-1-propanol der Umsetzung von Methylmercaptan mit Acrolein zugeführt wird.

Abweichungen in der Stöchiometrie von Methylmercaptan zu Acrolein können durch Messung der Mengenflüsse und durch die Bestimmung von nicht reagiertem Methylmercaptan und Acrolein in dem hergestellten 3-Methylthiopionaldehyd ermittelt werden. In einer Matrix von 3-Methylthiopropionaldehyd kann Acrolein auf Grund der charakteristischen Absorption bei 366 nm (durch die a,ß-ungesättigte Carbonylgruppe) mittels UV-Vis-Spektrometrie bestimmt werden. Diese Methode erlaubt eine zuverlässige Bestimmung von Acrolein mit ausreichender Genauigkeit für Acrolein-Konzentrationen von bis zu 0,6% in einer Matrix von 3-Methylthiopropionaldehyd. Bei höheren Acrolein-Konzentrationen sollte die Bestimmung von Acrolein mittels Gaschromatographie erfolgen. In einer Matrix von 3-Methylthiopropionaldehyd kann Methylmercaptan durch argentometrische Titration mit potentiometrischer Indikation des Endpunktes bestimmt werden.

Das erfindungsgemäße Verfahren ist bezüglich seiner Durchführung nicht auf eine Ausgestaltungsform limitiert. Denkbar sind sowohl eine kontinuierliche, eine semikontinuierliche als auch eine batch-weise Durchführung des Verfahrens. Bevorzugt wird allerdings eine kontinuierliche Betriebsweise. Denn bei einer kontinuierlichen Betriebsweise kann zum einen Acrolein und Methylmercaptan dem erfindungsgemäßen Verfahren aus ebenfalls kontinuierlich betriebenen Verfahren zugeführt werden und zum anderen ist die Einbindung in bereits bestehende oder geplante Verfahren zur Herstellung von Methionin möglich. Die Umsetzung von Methylmercaptan mit Acrolein zu 3-Methylthiopropionaldehyd kann in einem einzelnen oder in mehreren entweder parallel oder seriell angeordneten Reaktoren erfolgen. Bei einer vorzugsweise kontinuierlich betriebenen Verfahrensführung erfolgt die Umsetzung in einem kontinuierlich betriebenen Rührkessel oder in einem Strömungsrohr. Zur Erreichung möglichst vollständiger Umsätze wird die Umsetzung bevorzugt in zumindest zwei seriell angeordneten Reaktoren durchgeführt, insbesondere in einem kontinuierlich betriebenen Rührkessel als erstem Reaktor und einem Strömungsrohr als Nachreaktor. In dieser Kombination dient der kontinuierlich betriebene Rührkessel dazu einen möglichst hohen Umsatzgrad zu erzielen, und das anschließende Strömungsrohr dient zur Vervollständigung der Reaktion.

Hinsichtlich des Gehalts von Acrolein bzw. Methylmercaptan in den jeweiligen der Herstellung von 3-Methylthiopropionaldehyd zugeführten Strömen unterliegt das erfindungsgemäße Verfahren keinen Beschränkungen. Die jeweiligen Ströme enthalten daher Acrolein bzw. Methylmercaptan in Konzentrationen von 1 bis 99 Gew.-% oder sogar mehr. Außer Acrolein bzw. Methylmercaptan können die jeweiligen Ströme daher auch Nebenprodukte enthalten, sofern gewährleitet ist, dass die jeweiligen Ströme Acrolein und Methylmercaptan in ausreichenden Mengen enthalten, so dass Anlagenstillstände nicht eintreten können. Beispielsweise kann ein Acrolein enthaltender Strom auch Wasser und/oder Acetaldehyd enthalten, und ein Methylmercaptan enthaltender Strom kann auch Wasser, Dimethylsulfid und/oder Dimethylether enthalten.

Die Ausdrücke Menge und Mengen bezeichnen im Rahmen der vorliegenden Erfindung nicht Durchflussmengen sondern eine molare Menge der betreffenden Komponente bzw. molare Mengen der betreffenden Komponenten.

Der Umsetzung von Methylmercaptan mit Acrolein zu 3-Methylthiopropionaldehyd sowie der Bildung von 1,3-Bis(methylthio)-1-propanol schließen sich die Behandlung des aus den jeweiligen Umsetzungen erhaltenen Produktstromes in einem Strippungs-Schritt zur Entfernung von niedrigsiedenden Nebenkomponenten an, gefolgt von einer Destillation, bei der das Endprodukt im Vakuum als Kopfprodukt abdestilliert wird. Das Sumpfprodukt kann einer zweiten Destillationsstufe zugeführt werden, in der die höhersiedenden Rückstände durch Rückführung des Wertstoffs 3-Methylthiopropionaldehyd weiter aufkonzentriert werden.

Das im erfindungsgemäßen Verfahren gebildete bzw. dem erfindungsgemäßen Verfahren zugeführte 1,3-Bis(methylthio)-1-propanol liegt in der Regel nicht allein in Form dieser Verbindung vor. Denn 1,3-Bis(methylthio)-1-propanol ist ein sogenanntes Hemi- oder Halbthioacetal, das in einer Gleichgewichtsreaktion durch Addition von Methylmercaptan an der Carbonylfunktion des 3-Methylthiopropionaldehyds gebildet wird.

In einer Ausführungsform des erfindungsgemäßen Verfahrens liegt 1,3-Bis(methylthio)-1-propanol daher im Gleichgewicht zu 3-Methylthiopropionaldehyd und Methylmercaptan vor.

Das Gleichgewicht der Reaktion zur Bildung von 1,3-Bis(methylthio)-1-propanol aus 3-Methylthiopropionaldehyd und Methylmercaptan liegt jedoch stark auf der Seite von 1,3-Bis(methylthio)-1-propanol. Untersuchungen haben gezeigt, dass in einem Bereich von 10 bis 70°C der Anteil von 1,3-Bis(methylthio)-1-propanol in einem entsprechenden Gleichgewichtsgemisch zwischen mindestens 75% bei 70°C und mindestens 85% bei 10°C beträgt. Folglich liegt in einem Gleichgewichtsgemisch von 1,3-Bis(methylthio)-1-propanol der überwiegende Teil des darin enthaltenden Methylmercaptan in gebundener Form als Teil des Hemithioacetals von 3-Methylthiopropionaldehyd vor. Im Gegensatz zu Methylmercaptan dessen Lagerung ein Flüssiggaslager erfordert, kann 1,3-Bis(methylthio)-1-propanol in Reinform oder im Gemisch mit anderen Verbindungen, insbesondere mit 3-Methylthiopropionaldehyd, daher schon in einem einfachen Lagerbehälter und bei lediglich leichtem Überdruck gelagert werden. Die Lagerung von 1,3-Bis(methylthio)-1-propanol gestaltet sich deshalb deutlich einfacher, sicherer und kostengünstiger als die von Methylmercaptan.

Eine stöchiometrische Umsetzung von wasserfreiem Methylmercaptan mit wasserfreiem 3-Methylthiopropionaldehyd ergibt eine Lösung mit 31,6 Gew.-% Methylmercaptan, wobei 3-Methylthiopropionaldehyd und Methylmercaptan im Gleichgewicht zu 1,3-Bis(methylthio)-1-propanol vorliegen. Bei einem Verhältnis von Methylmercaptan zu 3-Methylthiopropionaldehyd von größer als 1:1 unterliegt das zusätzliche Methylmercaptan jedoch nicht mehr einer Additionsreaktion zum Hemithioacetal; vielmehr liegt das zusätzliche Methylmercaptan nur noch physikalisch gelöst vor und verursacht daher einen hohen Dampfdruck. Dadurch ergeben sich hohe Kosten für den zur Handhabung der Methylmercaptan enthaltenden Mischungen erforderlichen Druckbehälter und höhere Verluste an Methylmercaptan auf Grund der größeren im Abgas enthaltenen Mengen an Methylmercaptan.

Indem die Menge an Methylmercaptan in dieser Umsetzung erniedrigt wird, verringert sich auch der entsprechende Dampfdruck von Methylmercaptan in der erhaltenen Lösung. Dadurch werden die vorstehend genannten Nachteile entweder vermieden oder zumindest deutlich abgemildert. Allerdings vergrößert sich dann die Menge der bei der Herstellung von 3-Methylthiopropionaldehyd im Kreis zu fahrenden Verbindungen, was neben größeren Apparaten auch größere Energiemengen zum Heizen und Kühlen erfordert.

Es ist daher erstrebenswert, eine möglichst große Menge des zugeführten Methylmercaptans in das entsprechende Hemithioacetal des 3-Methylthiopropionaldehyds zu überführen und gleichzeitig die Menge des lediglich physikalisch gelösten Methylmercaptans in der erhaltenen Lösung so gering wie möglich zu halten. Im Rahmen der vorliegenden Erfindung wird dies für Lösungen von Methylmercaptan in 3-Methylthiopropionaldehyd mit einem Gehalt an Methylmercaptan von ca. 4,4 bis ca. 31,6 Gew.-% bei einem molaren Verhältnis von Methylmercaptan zu 3-Methylthiopropionaldehyd von ca. 0,1:1 (mol/mol) bis ca. 1:1 (mol/mol), bevorzugt 18,8 bis 31,6 Gew.-%, bei einem molaren Verhältnis von Methylmercaptan zu 3-Methylthiopropionaldehyd von ca. 0,5:1 (mol/mol) bis ca. 1:1 (mol/mol) oder 27,0 bis 29,3% Gew.-% bei einem molaren Verhältnis von Methylmercaptan zu 3-Methylthiopropionaldehyd von ca. 0,8:1 (mol/mol) bis ca. 0,9:1 (mol/mol), jeweils bezogen auf wasserfreies Methylmercaptan und wasserfreies 3-Methylthiopropionaldehyd.

In einer Ausführungsform des erfindungsgemäßen Verfahrens beträgt daher das molare Verhältnis von Methylmercaptan zu 3-Methylthiopropionaldehyd bei der Umsetzung zu 1,3-Bis(methylthio)-1-propanol 0,1:1 (mol/mol) bis 1:1 (mol/mol), besonders bevorzugt 0,5:1 (mol/mol) bis 1:1 (mol/mol) oder 0,8:1 (mol/mol) bis 0,9:1 (mol/mol).

Bei Vorliegen eines Überschusses von Methylmercaptan zu Acrolein kann die Abweichung in der Stöchiometrie dadurch ausgeglichen werden, dass das überschüssige Methylmercaptan mit 3-Methylthiopropionaldehyd zu 1,3-Bis(methylthio)-1-propanol umgesetzt wird. Alternativ kann bei Vorliegen eines Überschusses von Acrolein zu Methylmercaptan, die Abweichung in der Stöchiometrie dadurch ausgeglichen werden, dass 1,3-Bis(methylthio)-1-propanol der Umsetzung von Methylmercaptan mit Acrolein zugeführt wird. Bei dieser Variante reagiert das Acrolein aller Wahrscheinlichkeit nach nicht direkt mit 1,3-Bis(methylthio)-1-propanol sondern mit dem Gleichgewichtsanteil von Methylmercaptan, der im Gleichgewicht zum 1,3-Bis(methylthio)-1-propanol vorliegt. Das Methylmercaptan wird durch eine rasche Wiederherstellung des Gleichgewichts sehr schnell wieder nachgebildet. Aus diesen Gründen eignet sich 1,3-Bis(methylthio)-1-propanol sehr gut dazu, Acrolein durch Umsetzung zum 3-Methylthiopropionaldehyd abzupuffern, z.B. bei einem Stillstand der der Herstellung von 3-Methylthiopropionaldehyd vorgeschalteten Methylmercaptan-Herstellung.

In einer weiteren Ausführungsform des erfindungsgemäßen Verfahrens wird daher das überschüssige Acrolein mit 1,3-Bis(methylthio)-1-propanol zu 3-Methylthiopropionaldehyd umgesetzt.

Das im erfindungsgemäßen Verfahren zugeführte Methylmercaptan wird vorzugsweise durch die Umsetzung von Methanol mit einem Überschuss Schwefelwasserstoff in der Gasphase an einem mit Alkaliwolframat beladenen Aluminiumoxid hergestellt. Die weitere Aufarbeitung erfolgt durch dem Fachmann bekannten Schritte. Hinsichtlich der Aggregatform in der Methylmercaptan dem erfindungsgemäßen Verfahren zugeführt wird unterliegt das Verfahren keinen Einschränkungen. Das Methylmercaptan bzw. der Methylmercaptan enthaltende Strom kann daher im erfindungsgemäßen Verfahren sowohl eine Flüssig- als auch eine Gasphase sein. Im Vergleich zu einem gasförmiges Methylmercaptan enthaltenden Strom hat ein flüssiges Methylmercaptan enthaltender Strom den Vorteil, dass der Massenstrom genauer bestimmt werden kann und die nächste Verfahrensstufe präziser geregelt werden kann. Daher wird im erfindungsgemäßen Verfahren Methylmercaptan bevorzugt in flüssiger Phase eingesetzt.

Das zur Einstellung der Stöchiometrie im der Herstellung von 3-Methylthiopropionaldehyd erforderliche 1,3-Bis(methylthio)-1-propanol wird im einfachsten Fall aus dem unmittelbaren Verfahrensprodukt selbst generiert. Dies erfolgt in der Weise, dass ein Teil des Methylmercaptan enthaltenden Stroms nicht der Reaktion mit Acrolein zugeführt sondern vor der Umsetzung zu 3-Methylthiopropionaldehyd abgezweigt und der Bildung von 1,3-Bis(methylthio)-1-propanol durch Umsetzung von 3-Methylthiopropionaldehyd mit Methylmercaptan zugeführt wird.

In einer zusätzlichen Ausführungsform des erfindungsgemäßen Verfahrens wird daher ein Teil des Methylmercaptan enthaltenden Stroms vor der Umsetzung zu 3-Methylthiopropionaldehyd abgezweigt und der Bildung von 1,3-Bis(methylthio)-1-propanol durch Umsetzung von 3-Methylthiopropionaldehyd mit Methylmercaptan zugeführt.

Das auf diese Weise generierte 1,3-Bis(methylthio)-1-propanol kann dem erfindungsgemäßen Verfahren zugeführt werden, um Schwankungen in der Stöchiometrie von Acrolein zu Methylmercaptan in der Herstellung von 3-Methylthiopropionaldehyd aus Acrolein und Methylmercaptan auszugleichen. Zu diesem Zweck kann das 1,3-Bis(methylthio)-1-propanol zunächst zwischengespeichert werden, und das zwischengespeicherte 1,3-Bis(methylthio)-1-propanol zum Ausgleichen der Differenz zwischen Herstellung und Verbrauch von 1,3-Bis(methylthio)-1-propanol verwendet werden.

Parallel zu der Abzweigung eines Teils des Methylmercaptan enthaltenden Stroms vor der Umsetzung zu 3-Methylthiopropionaldehyd kann zusätzlich ein 1,3-Bis(methylthio)-1-propanol enthaltender Strom dem erfindungsgemäßen Verfahren zugeführt werden. Diese parallele Durchführung des erfindungsgemäßen Verfahrens ermöglicht ein schnelles und präzises Reagieren auf Schwankungen in der Stöchiometrie von Methylmercaptan zu Acrolein in der Umsetzung zu 3-Methylthiopropionaldehyd. Im Detail hat die Abzweigung eines Teils des Methylmercaptan enthaltenden Stroms aus dem erfindungsgemäßen Verfahren bzw. vor der Umsetzung zu 3-Methylthiopropionaldehyd den Vorteil, dass eine Abweichung in der Stöchiometrie von Methylmercaptan zu Acrolein bei einem schlagartig und/oder kurzfristig auftretenden Überschuss an zugeführtem Methylmercaptan gegenüber Acrolein zumindest schneller und präziser abgepuffert oder sogar unmittelbar bei Auftreten kompensiert wird. Die Zufuhr eines 1,3-Bis(methylthio)-1-propanol enthaltenden Stroms in das erfindungsgemäße Verfahren hat den Vorteil, dass einer Abweichung in der Stöchiometrie von Methylmercaptan zu Acrolein bei einem schlagartig und/oder kurzfristig auftretenden Überschuss an zugeführtem Acrolein gegenüber Methylmercaptan unmittelbar durch Umsetzung von überschüssigem Acrolein mit zugeführtem 1,3-Bis(methylthio)-1-propanol zu 3-Methylthiopropionaldehyd entgegengewirkt wird.

In einer weiteren Ausführungsform des erfindungsgemäßen Verfahrens wird daher ein Mindeststrom an Methylmercaptan vor der Umsetzung zu 3-Methylthiopropionaldehyd abgezweigt, und parallel dazu wird ein Mindeststrom an 1,3-Bis(methylthio)-1-propanol dem erfindungsgemäßen Verfahren zugeführt.

Vorzugsweise wird der vor der Umsetzung zu 3-Methylthiopropionaldehyd aus dem erfindungsgemäßen Verfahren abgezweigte Strom von Methylmercaptan der Bildung von 1,3-Bis(methylthio)-1-propanol durch Umsetzung von 3-Methylthiopropionaldehyd mit Methylmercaptan zugeführt.

Im Rahmen der vorliegenden Erfindung bezeichnet ein Mindeststrom an Methylmercaptan die Menge an Methylmercaptan, die zur Aufrechterhaltung der Umsetzung von Methylmercaptan mit 3-Methylthiopropionaldehyd technisch notwendig ist.

Im Rahmen der vorliegenden Erfindung bezeichnet ein Mindeststrom an 1,3-Bis(methylthio)-1-propanol die Menge an 1,3-Bis(methylthio)-1-propanol, die für eine schnelle und präzise Messung der technisch verfügbaren Flussregelung erforderlich ist,

Die Zwischenspeicherung des 1,3-Bis(methylthio)-1-propanols ermöglicht es, Schwankungen in der Stöchiometrie von Acrolein zu Methylmercaptan bei der Herstellung von 3-Methylthiopropionaldehyd zeitunabhängig und je nach Bedarf auszugleichen. Vorteilhafterweise wird die Menge des zwischengespeicherten 1,3-Bis(methylthio)-1-propanols an die Abweichungen in der Stöchiometrie von Methylmercaptan zu Acrolein angepasst. Das bedeutet, dass die Menge des zwischengespeicherten 1,3-Bis(methylthio)-1-propanols so groß sein sollte, dass, wenn sie dem erfindungsgemäßen Verfahren zugeführt wird, die Abweichung der Stöchiometrie, hervorgerufen durch die vorgeschalteten Anlagen, in einem in der Produktion üblichen Zeitraum ausgeglichen werden kann.

In einer weiteren Ausführungsform des erfindungsgemäßen Verfahrens wird daher das gebildete 1,3-Bis(methylthio)-1-propanol zwischengespeichert.

In einer bevorzugten Ausführungsform des erfindungsgemäßen Verfahrens ist daher die zuzuführende molare Menge des zwischengespeicherten 1,3-Bis(methylthio)-1-propanols mindestens so groß wie die Menge des überschussigen Acroleins.

Durch die Zwischenspeicherung von 1,3-Bis(methylthio)-1-propanol, insbesondere in einer zuzuführenden Menge, die mindestens so groß ist wie der Überschuss von Acrolein zu Methylmercaptan in der Herstellung von 3-Methylthiopropionaldehyd, wird sichergestellt dass eine Abweichung in der Stöchiometrie von Acrolein zu Methylmercaptan im erfindungsgemäßen Verfahren ausgeglichen werden kann.

Bei einem längeren Betrieb der 3-Methylthiopropionaldehyd-Herstellung mit einem Unterschuss an Methylmercaptan und Zuspeisung von 1,3-Bis(methylthio)-1-propanol sollte die Produktion an Methylmercaptan so stark erhöht werden, dass entweder keine oder nur eine kleine und/oder nur eine kurzfristige Abweichung in der Stöchiometrie von Acrolein zu Methylmercaptan auftritt. Andererseits sollte bei einem längeren Betrieb der 3-Methylthiopropionaldehyd-Herstellung mit einem Methylmercaptan-Überschuss unter gleichzeitiger Herstellung des 1,3-Bis(methylthio)-1-propanols die Produktion an Methylmercaptan entsprechend verringert werden.

In einer weiteren bevorzugten Ausführungsform des erfindungsgemäßen Verfahrens wird daher, wenn Acrolein im Überschuss zu Methylmercaptan vorliegt, das zwischengespeicherte 1,3-Bis(methylthio)-1-propanol der Umsetzung von Methylmercaptan mit Acrolein zugeführt und mit dem überschüssigen Acrolein zu 3-Methylthiopropionaldehyd umgesetzt.

Wenn in der Herstellung von 3-Methylthiopropionaldehyd aus Acrolein und Methylmercaptan das Acrolein im Überschuss zu Methylmercaptan vorliegt, gilt es Verluste von Acrolein zu vermeiden. Denn überschüssiges Acrolein polymerisiert sehr leicht zu hochsiedenden Rückständen. Da diese Polymerisation irreversibel erfolgt, steht die Menge von polymerisiertem Acrolein einer Umsetzung zu der Zielverbindung 3-Methylthiopropionaldehyd nicht mehr zur Verfügung. Dies führt zu einer verminderten Selektivität für die Bildung von 3-Methylthiopropionaldehyd bezogen auf die umgesetzte Menge an Acrolein. Sowohl in der Herstellung von 3-Methylthiopropionaldehyd als auch für die Herstellung von D,L-Methionin ist Acrolein der teuerste Ausgangsstoff. Jeder Verlust von Acrolein auf Grund der Bildung von Nebenprodukten ohne Wertschöpfung verschlechtert daher nicht nur unmittelbar die ökonomische Effizienz eines Verfahrens zur Herstellung von 3-Methylthiopropionaldehyd sondern insbesondere auch die eines Verfahrens zur Herstellung von D,L-Methionin. Wenn also Acrolein im Überschuss zu Methylmercaptan im erfindungsgemäßen Verfahren vorliegt, wird daher dem Verfahren 1,3-Bis(methylthio)-1-propanols in einer Menge zugeführt, die in jedem Falle ausreichend ist, um das überschüssige Acrolein zu 3-Methylthiopropionaldehyd. Die Bestimmung des 1,3-Bis(methylthio)-1-propanols erfolgt mit einer dem Fachmann bekannten und für diese Bestimmung geeigneten Analysemethode.

In einer Ausführungsform des erfindungsgemäßen Verfahrens ist daher die molare Menge des zugführten 1,3-Bis(methylthio)-1-propanols mindestens so groß ist wie der Überschuss an Acrolein.

Wird in dem erfindungsgemäßen Verfahren ein Produktgemisch erhalten, in dem nicht umgesetztes 1,3-Bis(methylthio)-1-propanol im Überschuss vorliegt, so würde dieses einen Verlust an den Wertstoffen Acrolein, Methylmercaptan und 3-Methylthiopropionaldehyd bedeuten. Daher sollte das nicht umgesetzte 1,3-Bis(methylthio)-1-propanol aus dem erfindungsgemäßen Verfahren erhaltene Produktgemisch wieder verwendet werden.

In einer bevorzugten Ausführungsform des erfindungsgemäßen Verfahrens wird daher das nicht umgesetzte 1,3-Bis(methylthio)-1-propanol zurückgewonnen.

Das zurückgewonnene 1,3-Bis(methylthio)-1-propanol kann entweder für eine spätere Umsetzung mit überschüssigem Acrolein zu 3-Methylmercaptopropionaldehyd zwischengespeichert werden oder direkt alternativ direkt mit dem überschüssigen Acrolein umgesetzt werden.

In einer bevorzugten Ausführungsform des erfindungsgemäßen Verfahrens wird das zurückgewonnene 1,3-Bis(methylthio)-1-propanol zwischengespeichert.

In einer alternativen bevorzugten Ausführungsform des erfindungsgemäßen Verfahrens wird, wenn Acrolein im Überschuss zu Methylmercaptan vorliegt, ein Strom mit überschüssigem Acrolein vor der Umsetzung zu 3-Methylthiopropionaldehyd abgezweigt und mit dem aus dem Produktgemisch abgetrennten nicht umgesetzten 1,3-Bis(methylthio)-1-propanol zu 3-Methylthiopropionaldeyhd umgesetzt.

In einer anderen alternativen bevorzugten Ausführungsform des erfindungsgemäßen Verfahrens werden gleichgroße Mengen von aus dem Produktgemisch abgetrennten nicht umgesetzten 1,3-Bis(methylthio)-1-propanol und Acrolein zu 3-Methylthiopropionaldhyd umgesetzt.

Im Rahmen der vorliegenden Erfindung hat sich gezeigt, dass sich die Zugabe eines Katalysatorgemisches aus mindestens einer stickstoffhaltigen Base und mindestens einer Säure positiv auf die Umsetzung von Methylmercaptan mit Acrolein zu 3-Methylmercaptan auswirkt: Die Bildung von höhersiedenden Verunreinigungen bzw. Rückständen wird durch Zugabe dieses Katalysatorgemisches merklich verringert und gleichzeitig die Ausbeute für die Zielverbindung erhöht.

Bevorzugt wird daher die Umsetzung zu 3-Methylthiopropionaldehyd in Gegenwart eines Katalysatorgemisches aus mindestens einer stickstoffhaltigen Base und mindestens einer Säure durchgeführt.

Bei einem leicht sauren pH-Wert ist das kurzfristig vorliegende Acrolein am stabilsten. Ein leicht saurer pH-Wert wird am einfachsten dadurch eingestellt, indem in dem bevorzugt eingesetzten Katalysatorgemisch die Säure im Überschuss zur Base vorliegt. Bevorzugt liegt daher in dem in einer Ausführungsform eingesetzten Katalysatorgemisch die Säure im Überschuss zur Base vor.

Die Zugabe des Katalysatorgemisches wirkt sich auch in der Umsetzung von Methylmercaptan mit 3-Methylthiopropionaldeyd zum 1,3-Bis(methylthio)-1-propanol positiv auf die Ausbeute für das Hemithioacetal und vermindert ebenfalls die Bildung von höher siedenden Rückständen.

Bevorzugt wird daher die Bildung von 1,3-Bis(methylthio)-1-propanol in Gegenwart eines Katalysatorgemisches aus mindestens einer stickstoffhaltigen Base und mindestens einer Säure durchgeführt.

Im Rahmen der vorliegenden Erfindung handelt es sich bei dem Katalysator um eine Mischung aus mindestens einer Base und mindestens einer Säure. Daher wird im Zusammenhang mit der vorliegenden Erfindung der erfindungsgemäß eingesetzte Katalysator gleichbedeutend mit Katalysatorgemisch verwendet. Durch die Kombination einer Base mit einer Säure im Katalysatorgemisch wird eine gute Löslichkeit dieses Gemisches in den beim erfindungsgemäßen Verfahren vorliegenden Flüssigphasen gewährleistet, so dass das Katalysatorgemisch in dem erfindungsgemäßen Verfahren als homogener Katalysator wirkt. Dies führt zu einer hohen Umsatzrate und einer hohen Selektivität für die Bildung sowohl von 3-Methylpropionaldehyd auch von 1,3-Bis(methylthio)-1-propanol.

Vorzugsweise erfolgt daher sowohl die Umsetzung von Methylmercaptan mit Acrolein zu 3-Methylthiopropionaldehyd als auch die Bildung von 1,3-Bis(methylthio)-1-propanol in Gegenwart eines Katalysatorgemisches aus mindestens einer stickstoffhaltigen Base und mindestens einer Säure.

Bevorzugt wird als Base eine unsubstituierte oder substituierte *N*-heterozyklische Verbindung oder ein Amin der Formel $NR^1R^2R^3$ verwendet, wobei $R^1$, $R^2$ und $R^3$ entweder identisch oder verschieden sind und abhängig voneinander Wasserstoff, einen Alkylrest mit ein bis vier Kohlenstoffatomen oder einen Arylalkylrest mit ein 7 bis 14 Kohlenstoffatomen bedeuten, mit der Maßgabe, dass wenn einer der Reste $R^1$, $R^2$ oder $R^3$ Wasserstoff ist, die beiden anderen Reste nicht Wasserstoff sind. Besonders bevorzugt handelt es sich bei der stickstoffhaltigen Base um Pyridin oder alkylsubstituiertes Pyridin, wie Picolin oder Lutidin, ein tertiäres Amin, wie Trimethylamin, Triethylamin, Tripropylamin, Tributylamin, Tridecylamin, Tridodecylamin oder Dimethylbenzylamin. Bei der Säure handelt es sich bevorzugt um eine Mineralsäure, insbesondere Salzsäure, Schwefelsäure oder Phosphorsäure, oder um eine organische Säure, insbesondere um Ameisensäure, Essigsäure, Propionsäure, Milchsäure, Bernsteinsäure, Weinsäure, Zitronensäure oder eine Mischung der vorstehend genannten Säuren.

Bevorzugt ist daher im erfindungsgemäßen Verfahren die Base ein substituiertes oder unsubstituiertes N-heterocyclisches Amin und/oder ein Amin der Formel $NR^1R^2R^3$, wobei $R^1$, $R^2$ und $R^3$ entweder identisch oder verschieden sind und abhängig voneinander Wasserstoff, einen Alkylrest mit ein bis vier Kohlenstoffatomen oder einen Arylalkylrest mit 7 bis 14 Kohlenstoffatomen bedeuten, mit der Maßgabe, dass wenn einer der Reste $R^1$, $R^2$ oder $R^3$ Wasserstoff ist, die beiden anderen Reste nicht Wasserstoff sind, und die Säure eine Mineralsäure und/oder eine organische Säure.

Bevorzugt ist die Base ein substituiertes oder unsubstituiertes *N*-heterocyclisches Amin und/oder ein Amin gemäß der vorstehenden Definition, insbesondere *N,N*-Dimethylbenzylamin und/oder Triethanolamin, und die Säure ist eine organische Säure, insbesondere Ameisensäure, Essigsäure, Propionsäure, Milchsäure, Bernsteinsäure, Weinsäure, Zitronensäure oder eine Mischung davon.

Im erfindungsgemäßen Verfahren begünstigen oder sogar katalysieren Metallkationen und insbesondere Schwermetallkationen wie $Fe^{2+}$ die Bildung von Hochsiedern aus Aldehyden. Zusätzlich zu der mindestens einen Base und der mindestens einen Säure enthält das Katalysatorgemisch daher bevorzugt auch einen Komplexbildner. Durch die Komplexierung von Metallkationen, insbesondere von Schwermetallkationen, wird die Bildung unerwünschter Hochsieder unterbunden, was sich positiv auf die Produktreinheit auswirkt. Hinsichtlich der zur Komplexierung von Metallkationen geeigneten Komplexbildner unterliegt das erfindungsgemäße Verfahren prinzipiell keinen Einschränkungen. Geeignete Komplexbildner sind im Rahmen der vorliegenden Erfindung grundsätzlich alle gängigen sogenannten mehrzähnigen Komplexbildner, also alle Komplexbildner mit mehr als einer zur Komplexbildung geeigneten funktionellen Gruppe wie einer Hydroxyl-, Amino- oder Carboxylgruppe. Im einfachsten Fall handelt es sich bei dem Komplexbildner um eine organische Säure mit mehr als einer Carboxylgruppe. Als Katalysatorgemisch wird daher bevorzugt eine stickstoffhaltige Base in Kombination mit mindestens einer Säure eingesetzt mit der Maßgabe, dass die zumindest eine der Säure eine komplexierende organische Säure ist. Im Rahmen der vorliegenden Erfindung handelt es sich bei der zur Komplexbildung geeigneten organischen Säure bevorzugt um eine zur Komplexierung von Metallionen wie $Fe^{2+}$ geeignete organische Säure, insbesondere um Weinsäure.

Bevorzugt enthält das im erfindungsgemäßen Verfahren eingesetzte Katalysatorgemisch daher zumindest eine komplexierende organische Säure.

Wie bereits vorstehend erläutert, wird 1,3-Bis(methylthio)-1-propanol in einer Gleichgewichtsreaktion aus 3-Methylthiopropionaldehyd und Methylmercaptan gebildet, wobei das Gleichgewicht dieser Reaktion für Temperaturen zwischen 10 und 70°C stark auf der Seite von 1,3-Bis(methylthio)-1-propanol liegt. Deshalb liegt der überwiegende Teil des darin enthaltenen Methylmercaptan in gebundener Form als Teil des Hemithioacetals von 3-Methylthiopropionaldehyd vor. Der geringere Anteil des nicht in gebundener Form vorliegenden Methylmeraptans ist in physikalisch gelöster Form im Produktgemisch der Bildung von 1,3-Bis(methylthio)-1-propanol enthalten. Die NMR-Spektren von Produktgemischen, die für einen bzw. sieben Tage gelagert wurden, zeigen hauptsächlich die entsprechenden Signale für 1,3-Bis(methylthio)-1-propanol (MMP-MC), 3-Methylthiopropionaldehyd (MMP) und Methylmercaptan (MC). Nebenprodukte werden in diesen NMR-Spektren - wenn überhaupt - nur in Spuren beobachtet. Durch Umsetzung mit Acrolein wird das in gebundener Form im 1,3-Bis(methylthio)-1-propanol enthaltene Methylmercaptan wieder freigesetzt und reagiert mit dem Acrolein unter Bildung von 3-Methylthiopropionaldehyd. Im Detail reagiert das Acrolein mit dem Gleichgewichtsanteil des nicht in gebundener Form vorliegenden Methylmercaptan. Das abreagierte "freie" Methylmercaptan wird aus der Gleichgewichtsreaktion kontinuierlich nachgebildet. Bei der Umsetzung von einem Mol 1,3-Bis(methylthio)-1-propanol mit einem Mol Acrolein werden zwei Mol 3-Methylthiopropionaldehyd gebildet. Folglich eignet sich 1,3-Bis(methylthio)-1-propanol sowohl zur Lagerung von Methylmeraptan als auch von 3-Methylthiopropionaldehyd.

Ein weiterer Gegenstand der vorliegenden Erfindung ist daher auch die Verwendung von 1,3-Bis(methylthio)-1-propanol als stabile Lagerform für Methylmercaptan und/oder 3-Methylthiopropionaldehyd.

Im Rahmen der vorliegenden Erfindung wird unter einer stabilen Lagerform für Methylmercaptan und/oder 3-Methylthiopropionaldehyd eine entsprechende Lagerform verstanden, in der Methylmercaptan und 3-Methylthiopropionaldehyd für zumindest 7 Tage ohne (nennenswerte) Bildung von Nebenprodukten gelagert werden kann.

Das erfindungsgemäß als Lagerform für Methylmercaptan und/oder 3-Methylthiopropionaldehyd eingesetzte 1,3-Bis(methylthio)-1-propanol kommt im einfachsten Fall aus dem erfindungsgemäßen Verfahren zur Herstellung von 3-Methylthiopropionaldehyd, in dem bei einem Überschuss von Methylmercaptan zu Acrolein das überschüssige Methylmercaptan mit 3-Methylthiopropionaldehyd zu 1,3-Bis(methylthio)-1-propanol umgesetzt wird. Alternativ oder ergänzend kann das als Lagerform für Methylmercaptan und/oder 3-Methylthiopropionaldehyd eingesetzte 1,3-Bis(methylthio)-1-propanol auch dem erfindungsgemäßen Verfahren zur Herstellung von 3-Methylthiopropionaldehyd zugeführt werden, wenn in diesem Verfahren Acrolein im Überschuss zu Methylmercaptan vorliegt. Denn dann wird das überschüssige Acrolein mit 1,3-Bis(methylthio)-1-propanol zu 3-Methylthiopropionaldehyd umgesetzt.

In einer Ausführungsform der erfindungsgemäßen Verwendung entstammt daher das 1,3-Bis(methylthio)-1-propanol dem erfindungsgemäßen Verfahren zur Herstellung von 3-Methylthiopropionaldehyd und/oder wird das 1,3-Bis(methylthio)-1-propanol dem erfindungsgemäßen Verfahren zur Herstellung von 3-Methylthiopropionaldehyd zugeführt.

**Beispiele:**

**A. Verwendete Methoden**

**1. Bestimmung von Methylmercaptan mittels argentometrischer Titration**

Verwendete Chemikalien:

- 2-Propanol (technisch)
- Natriumacetat in Ethanol (0,1 mol/l), Firma Bernd Kraft, Art.-Nr. 16590.3700
- Silbernitrat-Maßlösung (0,1 mol/l), Firma Sigma-Aldrich, Art.-Nr. 35375
- Stickstoff

Alternativ können auch Reagenzien anderer Hersteller mit vergleichbarer Qualität verwendet werden.

Verwendete Geräte:

- Titrando 907, Firma Metrohm, Art.-Nr. 2.907.0020
- Dosino 800, Firma Metrohm, Art.-Nr. 2.800.0020
- Tiamo™ (Titrationssoftware), Firma Metrohm, Art.-Nr. 6.6056.221
- Ag Tritrode mit Sulfid-Überzug, Firma Metrohm, Art.-Nr 6.0430.100S, mit 854 iConnect, Firma Metrohm, Art.-Nr. 1.854.0010
- Titrationgefäß (150 ml), Firma Metrohm, Art.-Nr. 6.1415.250, mit Deckel mit 5 Öffnungen, Firma Metrohm, Art.-Nr. 6.1414.010
- Haltering für Titrationsgefäß, Firma Metrohm, Art.-Nr. 6.2036.000
- Magnetrührer 801 mit Stativ, Firma Metrom, Art.-Nr. 2.801.0040
- Magnetrührstäbchen (Länge 25 mm), Firma Metrohm, Art.-Nr. 6.1903.030
- Magnetrührstabentferner
- Becherglas
- Spritzflasche
- Einwegspritzen (1 ml), Firma Luer
- Einwegkanülen (70 ml), Firma Luer
- Analysenwaage

Alternativ können auch andere Gerätekonfigurationen anderer Anbieter mit vergleichbaren Leistungsmerkmalen eingesetzt werden.

Es wurden 80 ml einer Natriumacetat-Lösung in Ethanol (0,1 mol/l) in dem Titrationsgefäß vorgelegt. Für ca. eine Minute wurde langsam Stickstoff in die vorgelegte Natriumacetat-Lösung eingeleitet. Während der Begasung der Natriumacetat-Lösung begast wurden mit einer Einwegspritze und einer Einwegkanüle 0,2 ml der zu bestimmenden Methylmercaptan-Probe aus der Probeflasche entnommen. Nach Beendigung der Stickstoffzufuhr wurde die Probe in das Titrationsgefäß gegeben und mittels Rückwägung die exakte Probenmenge bestimmt. Die Titration wurde mit Silbernitrat-Maßlösung (0,1 mol/l) durchgeführt und der Endpunkt mittels der Ag-Titrode mit Sulfid-Überzug bestimmt.

Wenn die Probe neben dem zu bestimmenden Methylmercaptan keinen Schwefelwasserstoff enthält, weist die Titrationskurve nur einen Wendepunkt in einem Potentialbereich zwischen -100 und +100 mV auf. Der Gehalt an Methylmercaptan in der Probenlösung (in Gew.-%) ergibt sich aus der Formel:

$$Gehalt\ MC = \frac{V(Maßlösung) * c(Maßlösung) * Molgewicht\,(MC) * 100}{Probeneinwaage}$$

Während der Titration fiel ein gelber Niederschlag von Silbermethylmercaptid aus. Bei Proben, die neben Methylmercaptan auch Schwefelwasserstoff enthielten, wurde die Anwesenheit von Schwefelwasserstoff durch einen schwarzen Niederschlag von Silbersulfid bei Zugabe von Silbernitrat angezeigt. In diesem Fall zeigte die Titrationskurve einen zweiten Wendepunkt in einem Potentialbereich zwischen +250 und +4550 mV. Der Gehalt an Methylmercaptan bzw. Schwefelwasserstoff in der Probe (in Gew.-%) ergibt sich aus den folgenden Formeln:

$$Gehalt\ MC = \frac{V(Maßlsg.,2.WP) - V(Maßlsg.,1.WP) * c(Maßlsg.) * Molgew.\,(MC) * 100}{Probeneinwaage}$$

$$Gehalt\ H2S = \frac{V(Maßlsg.,1.WP) * c(Maßlsg.) * Molgewicht(H2S) * 100}{Probeneinwaage * 2}$$

Maßlsg., 2. WP. und Maßlsg.,1. WP bedeuten die verbrauchten Volumina (in ml) der Silbernitrat-Maßlösung (mit der in mol/l angegebenen Konzentrationen), die zur Erreichung des zweiten bzw. ersten Wendepunktes in der Titrationskurve benötigt wurden. Die Probeneinwaage war in g und das Molgewicht wurde in g/mol eingetragen.

## 2. Bestimmung von Acrolein in 3-Methylthiopropionaldeyd mittels Photometrie

Verwendete Chemikalien:

- Demineralisiertes Wasser

Verwendete Geräte:

- UV-Vis-Photometer des Typs UV-1202 der Firma Shimadzu
- UV-Vis-Küvetten mit einer Schichtdicke von 10mm
- Transferpipette

Alternativ können auch andere Gerätekonfigurationen anderer Anbieter mit vergleichbaren Leistungsmerkmalen eingesetzt werden.

Vor der eigentlichen Bestimmung von Acrolein in 3-Methylthiopropionaldehyd wurde zunächst ein Abgleich gegenüber Wasser durchgeführt. Zu diesem Zweck wurde die UV-Vis-Küvette mit demineralisiertem Wasser befüllt und die so erhaltene Blindprobe wurde photometrisch vermessen. Im Anschluss wurde eine andere UV-Vis-Küvette zunächst durch mindestens einmaliges Spülen mit 3-Methylthiopropionaldheyd konditioniert. Dann wurde mit der Transferpipette ungefähr 1 ml der zu untersuchenden Probe in die UV-Vis-Küvette gegeben, verschlossen und photometrisch (bei einer Wellenlänge von 366 nm) vermessen. Zur Bestimmung des Gehalts von Acrolein in der Probe wurde die vom UV-Vis-Photometer gemessene Absorption mit einem Faktor 0,5220 multipliziert. Der Gehalt von Acrolein wurde dann von dem UV-Vis-Photometer mit einer Genauigkeit von drei Nachkommastellen in % angegeben. Mit dieser Methode lassen sich Konzentrationen von bis zu etwa 0,6 % Acrolein in einer 3-Methylpropionaldehyd enthaltenden Matrix mit ausreichender Genauigkeit bestimmen.

## 3. Bestimmung von *N,N*-Dimethylbenzylamin (DMBA) in 3-Methylthiopropionaldehyd mittels Titration

Verwendete Chemikalien:

- Elektrolytlösung für Ag/AgCl-Referenzsysteme c(KCl) = 3 mol/l (250 ml), Firma Metrohm, Art.-Nr. 6.2308.020
- Aufbewahrungslösung für kombinierte pH-Glaselektroden mit Referenzelektrolyt c(KCL) = 3 ml, Firma Metrohm, Art.-Nr. 6.2323.000
- Pufferlösung pH 4.00 (25°C) in Einwegportionsbeutel, Firma Metrohm, Art.-Nr. 6.2307.200
- Pufferlösung pH 7.00 (25°C) in Einwegportionsbeutel, Firma Metrohm, Art.-Nr. 6.2307.210
- Pufferlösung pH 9.00 (25°C) in Einwegportionsbeutel, Firma Metrohm, Art.-Nr. 6.2307.220
- Perchlorsäure in Eisessig (0,01 mol/l), Firma Bernd Kraft, Art.-Nr. 05101.3700
- Eisessig (ReagentPlus®), Firma Sigma-Aldrich, Art.-Nr. A6283
- 2-Propanol (Chromasolv®), Firma Sigma-Aldrich, Art.-Nr. 34863
- LiCl-Lösung, gesättigt in Ethanol

Alternativ können auch Chemikalien anderer Hersteller mit vergleichbarer Qualität eingesetzt werden.
Verwendete Geräte:

- Potentiometrischer Tirator Typ Titrando 907, Firma Metrohm, Art.-Nr. 2.907.0020
- Dosiersystem Typ Dosino 800, Firma Metrohm, Art.-Nr. 2.800.0020
- Tiamo™ (Titrationssoftware), Firma Metrohm, Art.-Nr. 6.6056.221
- Dosiereinheit Typ Dosing Unit 10 ml, Firma Metrohm, Art.-Nr. 6.3032.210
- Dosiereinheit Typ Dosing Unit 50 ml, Firma Metrohm, Art.-Nr. 6.3032.250
- Elektrode für Titration in nichtwässrigen Medien Typ Solvotrode, Firma Metrohm, Art.-Nr. 6.0229.100
- Titrationgefäß (150 ml), Firma Metrohm, Art.-Nr. 6.1415.250, mit Deckel mit 5 Öffnungen, Firma Metrohm, Art.-Nr. 6.1414.010
- Halterung für Titrationsgefäß, Firma Metrohm, Art.-Nr. 6.2036.000
- Magnetrührer mit Stativ Typ 801, Firma Metrom, Art.-Nr. 2.801.0040
- Magnetrührstäbchen (Länge 25 mm), Firma Metrohm, Art.-Nr. 6.1903.030
- Magnetrührstabentferner
- Becherglas
- Spritzflasche
- Einwegspritzen (1 ml), Firma Luer
- Einwegkanülen (70 ml), Firma Luer
- Analysenwaage

Alternativ kann auch eine andere Gerätekonfiguration mit vergleichbaren Leistungsmerkmalen eingesetzt werden.
Vor der ersten Messung wurde die pH-Elektrode mit Pufferlösungen mit einem pH von jeweils 4, 7 bzw. 9 kalibriert. Im Anschluss erfolgte die Bestimmung von DMBA in MMP als potentiometrische Titration. Ein Plastikbecher wurde mit ca. 50 ml Eisessig befüllt, anschließend wurden ca. 50 g Probe hinzugegeben, wobei die exakte Probenmenge mittels Wägung bestimmt wurde. Die Titration erfolgte mit Perchloressigsäure in Eisessig (0,01 mol/l), der Endpunkt wurde mittels einer Elektrode für Titration in nichtwässrigen Medien, z.B. Typ Solvotrode, Firma Metrohm detektiert.

$$Gehalt\ DMBA\ in\ MC\ [Gew.-\%] = \frac{V\left(Maß.Lsg,\ ml\right)*c\left(Maß.Lsg.,\frac{mol}{l}\right)*M\left(DMBA,\frac{g}{mol}\right)}{m\left(Probe,g\right)}$$

V. (Maß.Lsg., ml) entspricht dem verbrauchten Volumen an Perchloressigsäure-Maßlösung in ml.

c (Maß.Lsg., mol/l) entspricht der Konzentration der an Perchloressigsäure-Maßlösung in mol/l.

M (DMBA, g/mol) entspricht der Molmasse des zu bestimmenden DMBA (*N,N*-Dimethylbenzylamin) in g/mol.

m (Probe, g) entspricht den Einwaage der Probe in g.

**4. Rückstandsbestimmung mit einer Vakuum-Destillationseinheit**

Die Rückstandsbestimmung wird in einer Vakuum-Destillationseinheit durchgeführt (z.B. Kugelrohrverdampfer der Firma Büchi, Typ GKR-50)
Zur Bestimmung des Vorlagen-Leergewichts wurde das Gefäß für die zu destillierende Substanz gewogen.

Anschließend wurden 15 g der zu destillierende Flüssigkeit in die Vorlage eingewogen und die Destillationseinheit zusammengebaut. Druckregler der Vakuumpumpe wurde ein Druck von 30 mbar eingestellt. Die Heizung an der Destillationsvorlage auf eine Temperatur von 200°C eingestellt. Nach einer Zeit von 20 Minuten wurde die Destillation beendet. Nach dem Abkühlen der Destillationseinheit wurde die Apparatur belüftet, die Vakuumpumpe ausgeschaltet und die Glasteile auseinander gebaut. Die Destillationsvorlage wurde gewogen, und der Rückstand mit Hilfe der untenstehenden Formel bestimmt:

$$R\ddot{u}ckstand\ [Gew.-\%] = \frac{m\ (Vorlage, nach\ Dest.) - m(Vorlage, leer)}{m\ (Vorlage, vor\ Dest.)}$$

## 5. Gaschromatographie

Die gaschromatographischen Untersuchungen wurden mit einem Gaschromatographen des Typs HP 6890 der Firma Agilent durchgeführt, der mit einer Kapillarsäule des Typs 19091J-213 HP-5 5% Phenylmethylsiloxan von Agilent und einem Flammenionisationsdetektor ausgestattet war. Die Analyse wurde mittels Temperaturrampe von 40 auf 325°C mit einer Temperaturrate von 15°C pro Minute durchgeführt.

Im Rahmen der vorliegenden Erfindung wurde die Gaschromatographie - neben den anderen Methoden - zur Bestimmung von 3-Methylthiopropionaldehyd, Methylmercaptan und Acrolein eingesetzt. Das aus 3-Methylthiopropionaldehyd und Methylmercaptan gebildete 1,3-Bis(methylthio)-1-propanol war bei den in der Gaschromatographie angewandten Temperaturen nicht stabil und zerfällt daher bereits vor der Detektion in die Ausgangsverbindungen 3-Methylthiopropionaldehyd und Methylmercaptan.

## B. Versuche 1 bis 4:

25,16 g gestripptes 3-Methylthiopropionaldehyd (Methylmercaptopropionaldehyd MMP) (93,19 Gew.-%) aus industrieller Herstellung wurden in einem Kolben vorgelegt und mit einem Wasserbad temperiert. Die zur Bildung von 1,3-Bis(methylthio)-1-propanol (MMP-MC) erforderliche Menge Methylmercaptan (MC) (12,41 ml, 11,09 g) wurde in einem gekühlten Tropftrichter vorgelegt. Dem MMP der Versuche 2 und 4 wurden 0,027 g bzw. 0,095 einer Katalysatormischung, aus N,N-Dimethylbenzylamin (5,2 Gew.-% bezogen auf den Katalysator), Essigsäure, Weinsäure und Wasser, zugesetzt. Das Methylmercaptan wurde tropfenweise über den Tropftrichter zum 3-Methylthiopropionaldehyd über einen Zeitraum von ca. 7 bis 10,5 Minuten so hinzugegeben, dass eine Temperatur von ca. 25°C (Versuche 1 und 2) bzw. von ca. 40°C (Versuche 3 und 4) im Wesentlichen nicht überschritten wurde. Anschließend wurde die erhaltene Produktmischung noch für 15 Minuten bei 25°C (Versuche 1 und 2) bei 40°C (Versuche 3 und 4) gerührt.

Im Anschluss wurden die erhaltenen Produktgemische in einem Kolben mit Stickstoff überlagert für 1 Tag bzw. 7 Tage bei 15°C gelagert. In den NMR-Spektren der Produktgemische werden hauptsächlich die entsprechenden Signale für MMP-MC, MMP und MC beobachtet. Nebenprodukte sind allenfalls in Spuren zu beobachten.

## C. Versuche 5 bis 12:

Eine ausreichende Menge MMP (industriell hergestellt, 93,19 Gew.-%) wurde in einem Kolben vorgelegt. Zu dem MMP wurden aus Tropftrichtern 10,43 g (94,91 Gew.-%) des in den Versuchen 1 bis 4 hergestellten und einen Tag bzw. 7 Tage gelagerten MMP-MC und 3,76 (96,97 Gew.-%) Acrolein über einen Zeitraum von ca. 7,5 Minuten so zugetropft, dass eine Temperatur von ca. 60°C in der Flüssigphase im Kolben nicht überschritten wurde. Die Zugabe des Katalysators erfolgte gemäß den Angaben in der untenstehenden Tabelle. Anschließend wurden die Produktmische noch für 60 Minuten bei 60°C gerührt.

Das in Versuch 5 eingesetzte MMP-MC wurde bei 25°C hergestellt und für einen Tag bei 15°C gelagert. Die Zugabe des Katalysators erfolgte im zweiten Schritt (Herstellung von MMP aus MMP-MC und AC).

Das im Versuch 6 eingesetzte MMP-MC wurde bei 25°C hergestellt und für sieben Tage bei 15°C gelagert. Die Zugabe des Katalysators erfolgte im zweiten Schritt (Herstellung von MMP aus MMP-MC und AC).

Das im Versuch 7 eingesetzte MMP-MC wurde bei 25°C hergestellt und für einen Tag bei 15°C gelagert. Die Zugabe des Katalysators erfolgte im ersten Schritt (Herstellung von MMP-MC aus MMP und MC).

Das im Versuch 8 eingesetzte MMP-MC wurde bei 25°C hergestellt und für sieben Tage bei 15°C gelagert. Die Zugabe des Katalysators erfolgte im ersten Schritt (Herstellung von MMP-MC aus MMP und MC).

Das im Versuch 9 eingesetzte MMP-MC wurde bei 40°C hergestellt und für einen Tag bei 15°C gelagert. Die Zugabe des Katalysators erfolgte im zweiten Schritt (Herstellung von MMP aus MMP-MC und AC).

Das im Versuch 10 eingesetzte MMP-MC wurde bei 40°C hergestellt und für sieben Tage bei 15°C gelagert. Die Zugabe des Katalysators erfolgte im ersten Schritt (Herstellung von MMP-MC aus MMP und MC).

Das im Versuch 11 eingesetzte MMP-MC wurde bei 40°C hergestellt und für einen Tag bei 15°C gelagert. Die Zugabe des Katalysators erfolgte im ersten Schritt (Herstellung von MMP-MC aus MMP und MC).

Das im Versuch 12 eingesetzte MMP-MC wurde bei 40°C hergestellt und für sieben Tage bei 15°C gelagert. Die Zugabe des Katalysators erfolgte im ersten Schritt (Herstellung von MMP-MC aus MMP und MC).

Das Katalysatorgemisch enthielt 5,2 Gew.-% *N,N*-Dimethylbenzylamin.

**Tabelle 1:** Übersicht über die Versuche 1 bis 12.

| Versuch | Edukte | | Lagerung [d] | DMBA in Lsg.[ppm] | Kat. [g] | Rückstand [Gew.-%] |
|---|---|---|---|---|---|---|
| | MMP/MC | MMP-MC/AC | | | | |
| 1 | 1/1 | - | - | 89 | - | 0 |
| 2 | 1/1,08 | - | - | 129 | 0,027 | 0 |
| 3 | 1/1,04 | - | - | 92 | - | 0,01 |
| 4 | 1/1,06 | - | - | 232 | 0,095 | 0,01 |
| 5 | - | 1/0,91 | 1 | 143 | 0,026 | 0,1 |
| 6 | - | 1/1,05 | 7 | 147 | 0,026 | 0,08 |
| 7 | - | 1/1,03 | 1 | 113 | - | 0,13 |
| 8 | - | 1/1,03 | 7 | 113 | - | 0,05 |
| 9 | - | 1/1,02 | 1 | 150 | 0,028 | 0,01 |
| 10 | - | 1/1,03 | 7 | 151 | 0,028 | 0,01 |
| 11 | - | 1/1,03 | 1 | 151 | - | 0,01 |
| 12 | - | 1/1 | 7 | 149 | - | 0,03 |

Mit einer Einwaage von ca. 100 mg (Versuche 5 bis 12) wurden Gaschromatogramme der jeweiligen Produktgemische erstellt - mit *n*-Dodecan als internem Intensitätsstandard. Die Ergebnisse der Analytik sind in der nachfolgenden Tabelle zusammengestellt.

**Tabelle 2:** Übersicht über die Analytik für die Versuche 5 bis 12.

| Versuch | GC [Gew.-%] | | |
|---|---|---|---|
| | MMP | MC | AC |
| 5 | 92,42 | 0,50 | - |
| 6 | 94,66 | 0,03 | - |
| 7 | 93,88 | 0,15 | - |
| 8 | 93,90 | 0,12 | - |
| 9 | 94,11 | 0,16 | - |
| 10 | 95,67 | 0,14 | - |
| 11 | 94,55 | 0,02 | - |
| 12 | 94,60 | 0,04 | - |

Ferner wurden 15 g jedes Produktgemisches mit der Kugelrohrdestille destilliert. Dabei verblieb ein brauner Film von 0,01 bis 0,03 Gew.-% an der Kolbenwand, der als Dimer von MMP bestimmt werden konnte.

Die Versuche zeigen, dass unabhängig davon, ob das MMP-MC bei 25 oder 40°C hergestellt, ob es einen Tag oder sieben Tage gelagert wurde, und unabhängig davon, ob der Katalysator bei der MMP-MC oder bei der MMP-Herstellung hinzugegeben wird, keinen Einfluss auf die Bildung von hochsiedenden Rückständen hat.

**D. Versuch 13:**

In einem Reaktor wurden 90 Gewichtseinheiten MMP vorgelegt und dann wurden über einen Zeitraum von 24 Stunden 100 Gewichtseinheiten Acrolein (95,7 %) pro Stunde eingespeist. Gleichzeitig wurden über einen Zeitraum von 24 Stunden 83,5 Gewichtseinheiten Methylmercaptan (96,1 %) pro Stunde eingespeist. Zusätzlich wurden 0,02 Gewichtseinheiten N,N-Dimethylbenzylamin, gelöst in einem Überschuss Essigsäure, pro Stunde zugegeben. Zum Ausgleich des Unterschusses an Methylmercaptan wurden 28,5 Gewichtseinheiten einer Lösung von MMP/MMP-MC, die 7 Gewichtsprozent Methylmercaptan enthielt, eingespeist. Die Temperatur wurde bei 60°C gehalten. Es wurde die Menge an MMP ausgeschleust, die ein Ansteigen des Füllstands im Reaktor verursachte. Diese Menge wurde in einem Verweilzeitreaktor mit 0,5 Stunden Verweilzeit gepumpt und entsprechende wieder ausgeschleust. Die Reaktionslösung wurde in einer Stripkolonne von den leichtsiedenden Nebenprodukten der Acrolein- und Methylmercaptan-Herstellung befreit.

Die Analytik der so erhaltenen Reaktionslösung ergab, dass 206,1 Gewichtseinheiten MMP pro Stunde erhalten wurden. Eine Probe der Reaktionsmischung wurde bei 220°C und 30 mbar bis zur Gewichtskonstanz eingeengt. Es verblieb ein nicht verdampfbarer Rückstand von 0,3 Gew.-%. Eine Bestimmung der MMP-Konzentration ergab einen Gehalt von 97,1 %. Dies entspricht einer Ausbeute von mehr als 98%.

**Patentansprüche**

1. Verfahren zur Herstellung von 3-Methylthiopropionaldehyd durch Umsetzung von Methylmercaptan mit Acrolein **dadurch gekennzeichnet, dass** Abweichungen in der Stöchiometrie von Methylmercaptan zu Acrolein in der Umsetzung zu 3-Methylthiopropionaldehyd durch Zufuhr oder durch Bildung von 1,3-Bis(methylthio)-1-propanol ausgeglichen werden, wobei
   wenn Methylmercaptan im Überschuss zu Acrolein vorliegt, überschüssiges Methylmercaptan mit 3-Methylthiopropionaldehyd zu 1,3-Bis(methylthio)-1-propanol umgesetzt wird,
   oder
   wenn Acrolein im Überschuss zu Methylmercaptan vorliegt, 1,3-Bis(methylthio)-1-propanol der Umsetzung von Methylmercaptan mit Acrolein zugeführt wird.

2. Verfahren gemäß Anspruch 1, wobei das molare Verhältnis von Methylmercaptan zu 3-Methylthiopropionaldehyd bei der Umsetzung zu 1,3-Bis(methylthio)-1-propanol 0,1:1 (mol/mol) bis 1:1 (mol/mol) beträgt.

3. Verfahren gemäß Anspruch 1 oder 2, wobei das überschüssige Acrolein mit 1,3-Bis(methylthio)-1-propanol zu 3-Methylthiopropionaldehyd umgesetzt wird.

4. Verfahren gemäß einem beliebigen der Ansprüche 1 bis 3, wobei ein Teil des Methylmercaptan enthaltenden Stroms vor der Umsetzung zu 3-Methylthiopropionaldehyd abgezweigt und der Bildung von 1,3-Bis(methylthio)-1-propanol durch Umsetzung von 3-Methylthiopropionaldehyd mit Methylmercaptan zugeführt wird.

5. Verfahren gemäß einem beliebigen der Ansprüche 1 bis 4, wobei ein Mindeststrom an Methylmercaptan vor der Umsetzung zu 3-Methylthiopropionaldehyd abgezweigt wird und parallel dazu ein Mindeststrom an 1,3-Bis(methylthio)-1-propanol dem erfindungsgemäßen Verfahren zugeführt wird.

6. Verfahren gemäß einem beliebigen der Ansprüche 1 bis 5, wobei das gebildete 1,3-Bis(methylthio)-1-propanol zwischengespeichert wird.

7. Verfahren gemäß Anspruch 6, wobei die zuzuführende molare Menge des zwischengespeicherten 1,3-Bis(methylthio)-1-propanol mindestens so groß ist wie Menge des überschüssigen Acroleins.

8. Verfahren gemäß Anspruch 6 oder 7, wobei, wenn Acrolein im Überschuss zu Methylmercaptan vorliegt, das zwischengespeicherte 1,3-Bis(methylthio)-1-propanol der Umsetzung von Methylmercaptan mit Acrolein zugeführt und mit dem überschüssigen Acrolein zu 3-Methylthiopropionaldehyd umgesetzt wird.

9. Verfahren gemäß einem beliebigen der Ansprüche 1 bis 8, wobei die molare Menge des zugeführten 1,3-Bis(methylthio)-1-propanol mindestens so groß ist wie der Überschuss an Acrolein.

10. Verfahren gemäß Anspruch 8 oder 9, wobei nicht umgesetztes 1,3-Bis(methylthio)-1-propanol zurückgewonnen wird.

11. Verfahren gemäß Anspruch 10, wobei das zurückgewonnene 1,3-Bis(methylthio)-1-propanol zwischengespeichert wird.

12. Verfahren gemäß Anspruch 10, wobei, wenn Acrolein im Überschuss zu Methylmercaptan vorliegt, ein Strom mit überschüssigem Acrolein vor dem Umsetzung zu 3-Methylthiopropionaldehyd abgezweigt und mit dem aus dem Produktgemisch abgetrennten nicht umgesetzten 1,3-Bis(methylthio)-1-propanol zu 3-Methylthiopropionaldehyd umgesetzt wird.

13. Verfahren gemäß Anspruch 10, wobei gleichgroße Mengen von aus dem Produktgemisch abgetrennten nicht umgesetzten 1,3-Bis(methylthio)-1-propanol und Acrolein zu 3-Methylthiopropionaldhyd umgesetzt.

14. Verwendung von 1,3-Bis(methylthio)-1-propanol als stabile Lagerform für Methylmercaptan und/oder 3-Methylthiopropionaldehyd.

15. Verwendung gemäß Anspruch 14, wobei das 1,3-Bis(methylthio)-1-propanol dem Verfahren gemäß einem beliebigen der Ansprüche 1 bis 13 entstammt, und/oder das 1,3-Bis(methylthio)-1-propanol dem Verfahren gemäß einem beliebigen der Ansprüche 1 bis 13 zugeführt wird.

**Europäisches Patentamt**

**European Patent Office**

**Office européen des brevets**

# EUROPÄISCHER RECHERCHENBERICHT

Nummer der Anmeldung

EP 16 15 5700

| | EINSCHLÄGIGE DOKUMENTE | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X,D<br><br>Y | DE 16 18 879 B1 (SUMITOMO CHEMICAL CO)<br>16. Dezember 1971 (1971-12-16)<br>* Spalten 7,8: Vergleichsversuch * | 1<br><br>1-5,9,<br>10,12,13 | INV.<br>C07C319/18<br>C07C323/22 |
| X<br><br>Y | DE 11 68 408 B (INVENTA AG)<br>23. April 1964 (1964-04-23)<br>* Spalte 3, Zeilen 14-17 *<br>* Beispiele 1,2,4,5 * | 1,2<br><br>1-5,9,<br>10,12,13 | |
| X<br><br>Y | CH 253 951 A (MERCK & CO INC [US])<br>15. April 1948 (1948-04-15)<br>* Beispiele 1-4 * | 1<br><br>1-5,9,<br>10,12,13 | |
| X<br><br>Y | US 2 776 996 A (MADISON HUNT ET AL)<br>8. Januar 1957 (1957-01-08)<br>* Beispiele 1-9 * | 1<br><br>1-5,9,<br>10,12,13 | |
| X<br><br>Y | CH 582 665 A5 (DEGUSSA)<br>15. Dezember 1976 (1976-12-15)<br>* Beispiele 1-4 *<br>* Spalte 1, Zeilen 46-50 * | 1,3,9<br><br>1-5,9,<br>10,12,13 | **RECHERCHIERTE SACHGEBIETE (IPC)**<br><br>C07C |
| A,D | DE 16 18 884 B1 (SUMITOMO CHEMICAL CO)<br>16. September 1971 (1971-09-16)<br>* Spalte 2, Zeile 27 *<br>* Spalte 2, Zeilen 53-55 * | 1-15 | |

Der vorliegende Recherchenbericht wurde für alle Patentansprüche erstellt

| Recherchenort | Abschlußdatum der Recherche | Prüfer |
|---|---|---|
| Den Haag | 19. Juli 2016 | Fitz, Wolfgang |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 16 15 5700

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-07-2016

| Im Recherchenbericht angeführtes Patentdokument | | Datum der Veröffentlichung | Mitglied(er) der Patentfamilie | | | Datum der Veröffentlichung |
|---|---|---|---|---|---|---|
| DE 1618879 | B1 | 16-12-1971 | BE | 697178 | A | 02-10-1967 |
| | | | DE | 1618879 | B1 | 16-12-1971 |
| | | | ES | 339439 | A1 | 01-05-1968 |
| | | | GB | 1173174 | A | 03-12-1969 |
| | | | NL | 6705426 | A | 19-10-1967 |
| DE 1168408 | B | 23-04-1964 | KEINE | | | |
| CH 253951 | A | 15-04-1948 | KEINE | | | |
| US 2776996 | A | 08-01-1957 | KEINE | | | |
| CH 582665 | A5 | 15-12-1976 | AT | 331773 | B | 25-08-1976 |
| | | | BE | 813990 | A1 | 21-10-1974 |
| | | | CA | 1005460 | A | 15-02-1977 |
| | | | CH | 582665 | A5 | 15-12-1976 |
| | | | DD | 110862 | A5 | 12-01-1975 |
| | | | DE | 2320544 | B1 | 12-09-1974 |
| | | | ES | 423736 | A1 | 16-04-1976 |
| | | | FR | 2226393 | A1 | 15-11-1974 |
| | | | GB | 1400702 | A | 23-07-1975 |
| | | | IT | 1005995 | B | 30-09-1976 |
| | | | JP | S5012012 | A | 07-02-1975 |
| | | | NL | 7404691 | A | 23-10-1974 |
| | | | RO | 68025 | A | 30-12-1980 |
| | | | SE | 397344 | B | 31-10-1977 |
| | | | SU | 505357 | A3 | 28-02-1976 |
| | | | US | 4048232 | A | 13-09-1977 |
| DE 1618884 | B1 | 16-09-1971 | BE | 697532 | A | 02-10-1967 |
| | | | DE | 1618884 | B1 | 16-09-1971 |
| | | | ES | 339752 | A1 | 16-08-1968 |
| | | | ES | 352807 | A1 | 01-09-1969 |
| | | | GB | 1150252 | A | 30-04-1969 |
| | | | NL | 6705792 | A | 26-10-1967 |
| | | | YU | 83467 | A | 30-06-1975 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1618884 A
- DE 1618879 A
- DE 1618889 A